Europäisches Patentamt

European Patent Office

Office européen des brevets

(19)

(11) Veröffentlichungsnummer: **0 260 222 B1**

# EUROPÄISCHE PATENTSCHRIFT

(12)

(45) Veröffentlichungstag der Patentschrift: **11.09.91**

(51) Int. Cl.⁵: **A61B 17/58**

(21) Anmeldenummer: **87810411.6**

(22) Anmeldetag: **21.07.87**

(54) Osteosyntheses Implantatset.

(30) Priorität: **05.08.86 CH 3152/86**

(43) Veröffentlichungstag der Anmeldung:
**16.03.88 Patentblatt 88/11**

(45) Bekanntmachung des Hinweises auf die
Patenterteilung:
**11.09.91 Patentblatt 91/37**

(84) Benannte Vertragsstaaten:
**AT BE CH DE ES FR GB IT LI NL SE**

(56) Entgegenhaltungen:
**EP-A- 0 011 528**
**EP-A- 0 159 502**
**DE-A- 3 509 417**
**US-A- 3 739 773**
**US-A- 4 329 743**

(73) Patentinhaber: **Synthes AG Chur**
**Grabenstrasse 15**
**CH-7002 Chur(CH)**

(72) Erfinder: **Illi, Oscar Emil, Dr. med.**
**Einhardweg 2**
**CH-8603 Schwerzenbach(CH)**

(74) Vertreter: **Lusuardi, Werther Giovanni, Dr.**
**Dr. Lusuardi AG, Kreuzbühlstrasse 8**
**CH-8008 Zürich(CH)**

## Beschreibung

Die vorliegende Erfindung betrifft ein osteosynthetisches Implantatset zur Fixation und oder Abstützung von Fragmenten von Deck- und Röhrenknochen, insbesondere bei Kindern.

Die Osteosynthese wird heute vorwiegend bei Erwachsenen angewendet. Nur in einer sehr begrenzten Zahl von Fällen, beispielsweise bei komplexen Frakturen, kommt die Osteosynthese auch bei Kindern im Wachstumsalter zum Einsatz. Bekanntlich kann die Verwendung von osteosynthetischen Implantaten bei Kindern zu Wachstumsstörungen, die entsprechende operative Korrekturen bedingen.

Es wurden erste Versuche unternommen, die zwar nicht auf die Kinderchirurgie spezifisch durchgeführt wurden, um mit Verbindungselementen, insbesondere Schrauben, aus resorbierbarem Material zu arbeiten. Die dabei gemachten Erfahrungen waren bezüglich der Anwendung jedoch nicht ermutigend.

Solche biokompatible, resorbierbare Materialien sind an sich bekannt. Die US-PS-3.739.773 beschreibt die Herstellung eines biokompatiblen, resorbierbaren Materials (Polyglykol-Säure) sowie daraus herstellbare feste Teile sowie auch Fasern und daraus hergestellte gewobene Teile. Es wird beispielsweise ein gewobenes Rohrstück zum Verbinden einer natürlichen Arterie mit einer künstlichen Arterie beschrieben.

Die EP-A-00 11 528 beschreibt ein Herstellungsverfahren für ein osteosynthetisches, biokompatibles resorbierbares Material und daraus hergestellte feste Teile wie Platten, Nägel oder Schrauben.

Zum einen war der Anwendungsbereich sehr beschränkt, zum anderen hielten die Schrauben den Belastungen beim Zusammenschrauben nicht Stand. Meist brachen die Schraubenköpfe ab, weil das resorbierbare Material eine zu geringe Festigkeit aufweist. Auch war damit das Problem der Fixation in jenen Fällen nicht gelöst, bei denen die einzelnen Fragmente noch mit zusätzlichen Elementen wie Platten oder Cerclagen gehalten werden müssen.

Bisher ging man allerdings von den Ueberlegungen und Erfahrungen der Osteosynthese bei Erwachsenen aus. Unter Verwendung von Metallimplantaten musste und wurde eine sogenannte Mikrostabilität, das heisst eine Stabilität, die nur noch kleinste Bewegungen zwischen den Fragmenten zulässt, erstrebt. Mikrostabile Verbindungen lassen Belastungen von ca. 60-80kp über den Frakturbereich zu, ohne dass dabei wahrnehmbare Bewegungen feststellbar sind.

Die erheblich elastischeren Knochen von Kindern und Jugendlichen im Wachstumsalter gestatten jedoch eine sogenannte Makrostabilität. Hierunter werden Verbindungen verstanden, die bei einer Belastung von 10-20kp über den Frakturbereich noch geringe, wahrnehmbare Bewegungen zulassen. Die Fraktur ist somit lagerungs- und bewegungsstabil. Eine belastungsstabile Osteosynthese (vgl. Erwachsenenchirurgie) wird nicht angestrebt.

Diese Erkenntnis wird vom Erfinder bewusst ausgenützt, um unter Verwendung bekannter Materialien zu völlig neuen Osteosynthese - Implantaten zu gelangen, die für die Kinderchirurgie geeignet sind..

Die bekannten nicht biodegradablen Implantatset sind vielfach auch mit Schrauben ausgerüstet. Eine solche Schraube ist beispielsweise aus der EP-A-0 172 130 bekannt geworden. Es handelt sich hierbei um eine biokompatible Senkkopfschraube, die mit einem hohlzylindrischen Schaft versehen ist. Im Bereich des Schraubenkopfes ist diese Durchgangsbohrung mit nach aussen gericheteten Erweiterungen zu zwei Innensechskantaufnahmen mit unterschiedlichem Innenmass geformt. Für die Verwendung mit dem erfindungsgemässen Band und zur Fertigung aus biodegradablem Material ist eine solche Schraube nicht geeignet.

Es ist die Aufgabe der vorliegenden Erfindung, ein Implantatset aus verschiedenen Implantaten für die Kinderchirurgie zu schaffen, mit welchem es möglich ist, osteosynthetische Eingriffe durchzuführen, die keine oder nur geringe Wachstumsstörungen hervorrufen.

Diese Aufgabe löst die Erfindung gemäss Oberbegriff des Patentanspruchs, welches die Merkmale des kennzeichnenden Teils des Patentanspruchs aufweist. Die Verwendung von Verbindungselementen aus resorbierbarem Material mit ebensolchen gewobenen oder gewirkten Bändern, die als Zuggurten wirken, macht es möglich, die Osteosynthese auch bei Kindern im Wachstumsalter zu verwenden, ohne dass Wachstumsstörungen auftreten, wobei die oben beschriebene, erforderliche Makrostabilität erreicht wird.

Bei Röhrenknochenfrakturen verwendet man vorteilhaft hohlzylindrische Schrauben mit einer Durchgangsbohrung am Schaftende, die aus biodegradablem, biokompatiblem Material hergestellt sind, die einen flachen Kopf aufweisen und einen hohlzylindrischen Schaft mit radial nach aussen gerichteten über die volle Länge des hohlzylindrischen Schaftes sich erstreckende Erweiterungen in der Form von Längsnuten haben. Gestaltet man die Erweiterungen zudem noch als Kehlnuten, so wird auch vermieden, dass eine radial nach aussen gerichtete Kraft auftritt, wodurch auch keine Sprengung der Schraube auftreten kann.

Die gewebemässige Durchwachsung sowie die Resorption des resorbierbaren Material lässt sich verbessern, wenn die Hohlschrauben mit radial ausgerichteten Durchgangsbohrungen versehen werden.

Das für sich bekannte Werkzeug zur Verwendung der beschriebenen Schrauben hat eine Aussenkontur, die der Form des Hohlraumes der Schrauben entspricht und einen achsialen, verschiebbaren Zentrierstift aufweist. Der Zentrierstift dient der Zuführung der Schraube fluchtend mit dem Bohrloch im Knochen und um die verwendeten Bänder zu durchbohren.

Es zeigt

Figur 1    ein Beispiel craniofacialer Anwendung des erfindungsgemässen Implantatsets;

Figur 2    ein Band mit Verbindungselementen aus resorbierbarem Material;

Figur 3    eine Buchse und einen Dübel in perspektivischer Darstellung im Schnitt;

Figur 4    zeigt die Anwendung des Implantatsets bei einer Röhrenknochenfraktur bei der

Figur 5    Hohlschrauben zum Einsatz kommen (Seitenansicht);

Figur 6    zeigt einen entsprechenden Schraubenzieher in perspektivischer Darstellung und

Figur 7    einen Teilschnitt durch denselben. In

Figur 8    ist die Festigkeit der Verbindung zwischen den Fragmenten graphisch in Abhängigkeit der Zeit dargestellt.

Je nach Art der zu verbindenden Knochenfragmente werden verschiedene Verbindungselemente verwendet. Diese lassen sich aus bekannten Materialien, wie z.B. Polygluconat, Polylactat etc., herstellen. Diese Materialien sind biodegradabel und biokompatibel.

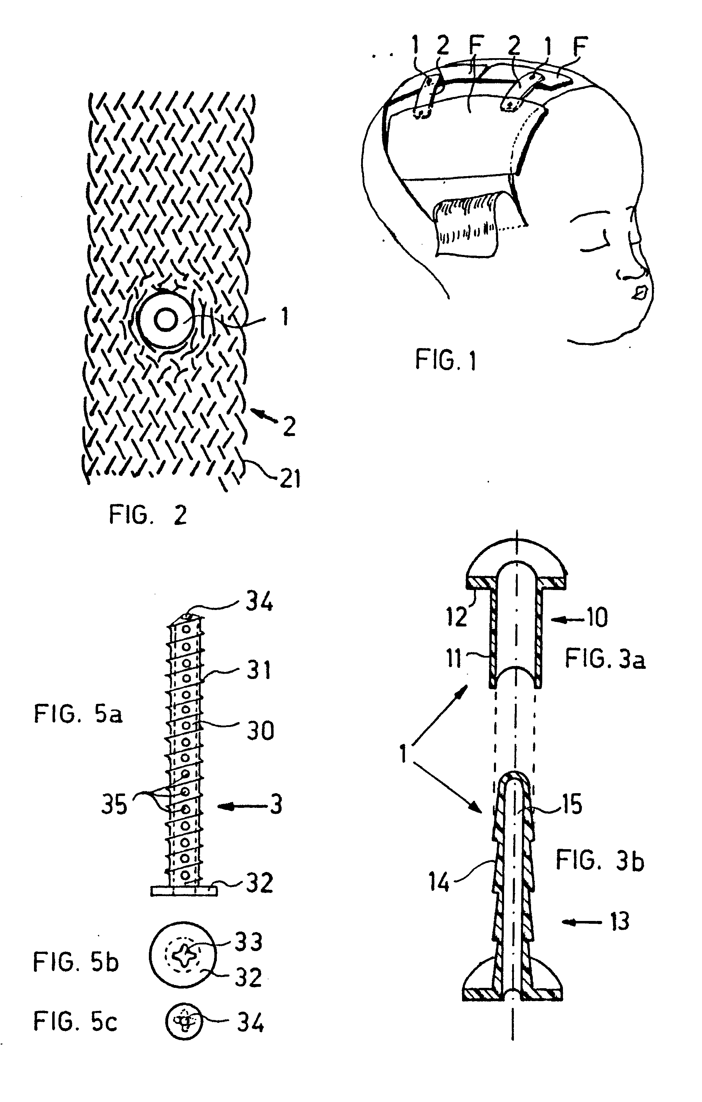

In Figur 3a und b sind Verbindungselemente für die Verbindung von Plattenknochenfragmenten in perspektivischer Ansicht im Schnitt gezeigt. Mit 10 ist eine Stopfbuchse bezeichnet, die einen rohrförmigen Teil 11 mit einem endständigen ringförmigen Kragen 11 aufweist. Die Wandstärke beträgt beispielsweise 0,5 mm bei einer Gesamtlänge von ca. 2 mm. Der Durchmesser des Kragens kann ungefähr 5 mm betragen, während der Innendurchmesser des rohrförmigen Teils 11 ca. 2 mm gross ist. In der Grösse angepasst ist das Gegenstück 13 in der Form eines Dübels oder Stopfens, der ebenfalls einen Kragen 16 hat. Die sägezahnförmige Aussenkontur 14 dient dazu, einen guten Presssitz zu bewerkstelligen, ohne dass die Buchse 10 oder der Stopfen 13 beim Zusammenstecken bricht. Das zentrale, zylindrische Sackloch 15 dient zur Halterung des Stopfens oder Dübels auf einem chirurgischen Instrument, beispielsweise einer speziellen Parallelzange, die das Zusammenstecken erleichtert. Die Länge des Stopfens oder Dübels ist vorzugsweise grösser als die Länge der Buchse 10, weil die Buchse meist kürzer als die Plattenknochendicke sein sollte. Es ist daher auch durchaus möglich, mit Schrauben und Muttern zu arbeiten, wo die Zugänglichkeit dies erlaubt.

Während bei Deckknochenverbindungen oftmals die zur Fixierung verwendeten Bänder nur einseitig angebracht werden können, ist es bei Röhrenknochen möglich und wünschenswert, an zwei gegenüberliegenden Seiten des Knochens die Bänder 3 anzubringen. Die verwendeten Verbindungselemente 1 durchbohren die Bänder 2. Die Bänder wirken so vergleichbar mit Zuggurten.

Für eine solche Fragmentteilverbindung verwendet man Hohlschrauben 3 als Verbindungselemente. Eine solche Schraube 3 ist in Figur 5a - c dargestellt. Sie umfasst einen Schaft 30, der auf seiner gesamten Länge ein Aussengewinde 31 mit relativ grossem Gewindegangsabstand hat. Der Schraubenkopf 32 ist vorzugsweise flach und hat die Form eines Kragens. Die Hohlschraube ist annähernd auf ihrer gesamten Länge von einem Hohlraum 33 durchsetzt. Dieser kann verschieden gestaltet sein. Wichtig ist nur, dass er über die gesamte Länge sich erstreckende, radial nach aussen gerichtete Erweiterungen hat. Im dargestellten Beispiel ist der Hohlraum im Querschnitt kreuzförmig gestaltet. Die Schraubenspitze ist von einer achsial verlaufenden Durchgangsbohrung 34 durchsetzt, deren Bedeutung nachfolgend noch beschrieben werden wird. Mehrere radiale Bohrungen 35, die in den inneren Hohlraum 33 münden, dienen dazu, die gewebemässige Durchwachsung zu fördern und den biologischen Abbau der Schraube zu erleichtern.

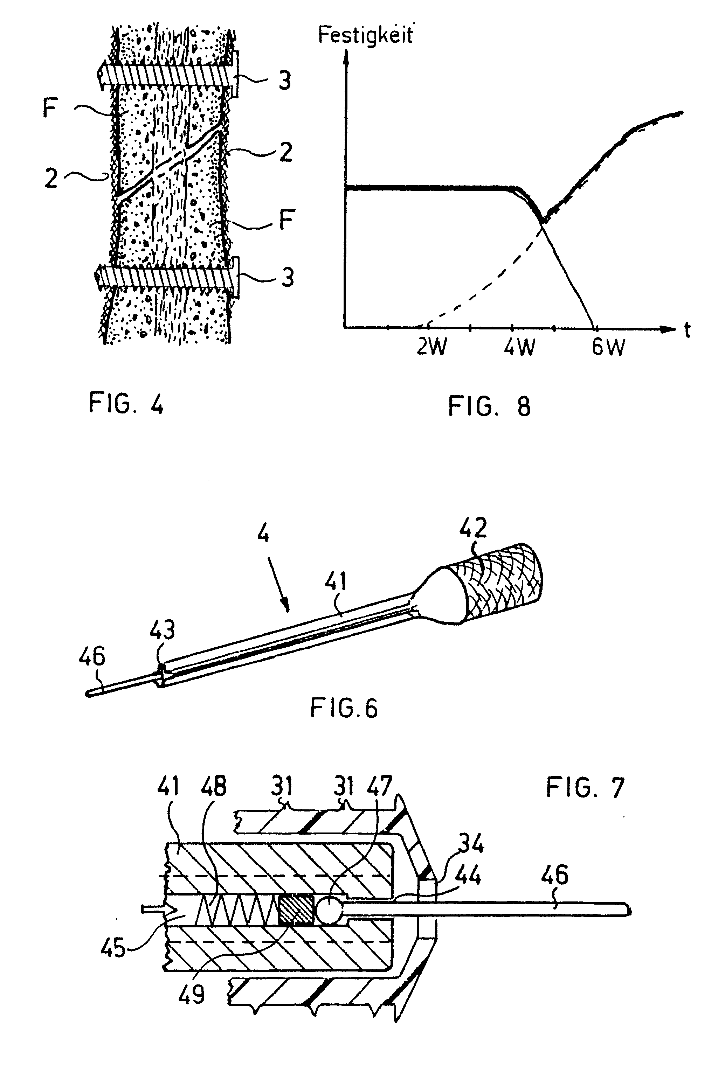

Um die Hohlschraube einzuschrauben, ist ein entsprechender Schraubenzieher erforderlich. Dieser gleicht einem Imbusschlüssel mit einem Querschnitt entsprechend der Kontur des Hohlraumes 33 der Schraube. Das Werkzeug 4 ist in Figur 6 gesamthaft und in Figur 7 teilweise im Schnitt dargestellt. Es besteht aus einem im Querschnitt, hier kreuzförmigen, hohlen Schaft 41 mit einem als Griff 42 dienenden, verdickten Ende. Der Griff 42 ist abschraubbar. An der Schaftspitze ist eine Bohrung 44, durch die eine im zylindrischen Hohlraum 45 des Schaftes 41 beweglich gelagerte Zentrierspitze 46 herausragt. Die Zentrierspitze 46 ist rückwärtig verdickt zu einem Führungsteil 47, der in der zylindrischen Bohrung 45 lagert. Eine schraubenförmige Druckfeder 48 mit Druckplättchen 49 schiebt die Zentrierspitze in ihre normale Endlage. Rückwärtig findet die Feder 48 am abschraubbaren Griff 42 ihren Anschlag.

Die Zentrierspitze 46 dient beim Zusammenschrauben als Führung. Die Schraube 3 wird auf den Schraubenzieher 4 aufgesetzt. Dabei überragt die Zentrierspitze 46 die Schraube 3 durch dessen Durchgangsbohrung 34 an der Spitze.

Nun kann man mit der Zentrierspitze 46 durch die Maschen des Bandes 2 in die bereits vorbereitete Bohrung im Knochen stossen und den Weg für die Schraube 3 vorbahnen. Auf der Gegenseite wird wiederum das Bohrloch mit der Spitze ertastet und das Gewebe des Bandes 2 durchbohrt. Da die Spitze einfedern kann, ist die Gefahr vermieden, dass die Zentrierspitze 46 Verletzungen bewirkt.

Die kreuzförmige Aussenkontur des Schraubenzieherschaftes 41, welche genau dem Hohlraum 33 der Schraube 3 entspricht, vermittelt auch bei einem relativ hohen Drehmoment einen geringen Flächendruck in der Schraube 3 und vermeidet eine radiale Kraft, die eine Sprengung verursachen könnte. Die Kraft muss daher nicht mehr über den Schraubenkopf 32 auf den Schraubenschaft 30 übertragen werden. Die Gefahr einer Zerstörung ist somit erheblich vermindert. Tritt ein solcher Bruch dennoch auf, so ist der Eingriff zwischen den noch verbleibenden Teilen und dem Schraubenzieherschaft gleichwohl erhalten und die Teile können wieder herausgeschraubt werden.

Schliesslich sei noch kurz über den Ablauf des Heilprozesses bei der Verwendung der neuen Implantate gesprochen. Hierzu wird auf die graphische Darstellung gemäss Figur 8 verwiesen. Auf der Abszisse des Koordinatensystems ist die Zeit abgetragen, während die Ordinate die Festigkeit der Verbindung zwischen den verbundenen Knochenfragmenten wiedergibt. Der Verlauf der Kurven ist lediglich qualitativ zu betrachten und nicht quantitativ. Als Zeitpunkt 0 wird der Abschluss des operativen Eingriffs angesehen. Die Festigkeit der Verbindung entspricht anfänglich der Festigkeit der verwendeten Implantate. Diese ist als dünne ausgezogene Linie dargestellt. Ungefähr nach vier Wochen beginnt der biologische Abbau des resorbierbaren Materials, dessen Festigkeit dann stark abnimmt. Etwa nach zwei Wochen beginnt jedoch bereits die bindegewebemässige Durchwachsung. Die Festigkeit durch die Verwachsung ist strichliniert gezeichnet. Ab der vierten bis sechsten Woche ca. beginnt die Verknöcherung, so dass dann die Festigkeit über die Festigkeit der Implantatverbindung steigt. Die Gesamtfestigkeit entspricht jedoch nicht der Summierung beider Festigkeitswerte, sondern wird durch die Hüllkurve repräsentiert, die ausgezogen, dick gezeichnet ist.

Bei der relativ kurzen Zeit bis zum Beginn des Abbaus des resorbierbaren Materials dürfte es kaum zu Wachstumsstörungen Anlass geben.

Ein Zweiteingriff zur Entfernung der Implantate entfällt. Damit wird, beim kindlichen Skelett, ein zusätzlicher Wachstumsreiz, der durch die Operation und Freilegung des Knochens bedingt ist, vermieden.

Insgesamt wird so die Krankheitsdauer aber auch die Gesamtkosten des/der Spitalbehandlung erheblich gesenkt.

In der Figur 2 ist das Band 2 gewoben dargestellt. Die relativ lockere Webung erleichtert das Durchstossen der Verbindungselemente durch das Band 2. Damit die Bänder sich beim Abschneiden nicht auftrennen, ist es wichtig, die Schussfäden mit den Längsfäden zu verschweissen.

**Patentansprüche**

1. Ostheosyntheses Implantatset zur makrostabilen Fixation und/oder Abstützung von Fragmenten von Deck-oder Röhrenknochen, insbesondere bei Kindern, bestehend aus mindestens einem Zuggurtband (2), welches Kräfte zu übertragen im Stande ist und aus resorbierbarem, biokompatiblen Material hergestellt ist, und aus biokompatiblen Verbindungselementen (3, 10, 13) zur Befestigung des Zuggurtbandes an den zu fixierenden Knochenelementen, dadurch gekennzeichnet, daß das Band (2) so gewirkt oder gewoben ist, daß die Fäden (21) sich unter einem Winkel von annähernd 60° kreuzen und die Schußfäden in regelmäßigen Abständen mit den Längsfäden verschweißt sind, wodurch die elastische Verformbarkeit des Bandes (2) in Längsrichtung gering ist, und ein leichtes Durchbohren oder Durchstechen beim Anbringen der Befestigungselemente (3, 10, 13) erlaubt wird.

2. Implantatset nach Anspruch 1, dadurch gekennzeichnet. dass das Verbindungselement aus einem biodegradablen und biokompatiblen Material hergestellt ist und zwei Teile umfasst, nämlich eine Buchse (10) mit einem rohrförmigen Teil (11), einem Kragen (12) und einen hohl (15) ausgebildeten, einseitig geschlossenen und am offenen Ende mit einem Kragen (16) versehenen Dübel (13), dessen Aussenwand sägezahnförmige Erhebungen (14) aufweist, die so dimensioniert sind, dass der Dübel haftend in den rohrförmigen Teil der Buchse einschiebbar ist.

3. Implantatset nach Anspruch 1, wobei das Verbindungselement eine hohlzylindrische Schraube (3) mit einer Durchgangsbohrung am Schaftende ist, die aus biodegradablem, biokompatiblem Material hergestellt ist, dadurch gekennzeichnet, dass sie einen flachen Kopf (32) aufweist und einen hohlzylindrischen Schaft (30) mit radial nach aussen gerichteten über die volle Länge des hohlzylindrischen Schaftes sich erstreckende Erweiterungen (33) in der Form von Längsnuten hat, und dass der hohlzylindrische Schraubenschaft mit radial ausgerichteten Durchgangsbohrungen (35) versehen ist.

## Claims

1. Osteosynthetic implant set for the macro stable fixing and/or supporting of fragments in cover or tubular bones, particularly in children, comprising at least one tension chord band 2, which is able to transfer forces and which is made from resorbable, biocompatible material, and biocompatible connecting elements (3, 10, 13) for fixing said band to the bone element to be fixed, characterized in that the band (2) is so knitted or woven that the threads (21) cross one another under an angle of approximately 60° and the weft threads are sealed at regular intervals to the longitudinal threads, so that the elastic deformability of the band (2) in the longitudinal direction is limited and an easy passing or piercing through during the fitting of the fixing elements (3, 10, 13) is possible.

2. Implant set according to claim 1, characterized in that the connecting element is made from biodegradable, biocompatible material and has two parts, namely a bush (10) with a tubular part (11), a collar (12) and a hollow (15) dowel (13) closed at one side and provided at the open end with a collar (16), whose outer wall has sawtooth-like projections (14), which are so dimensioned that the dowel can be adhesively inserted in the tubular part of the bush.

3. Implant set according to claim 1, in which the connecting element is a hollow cylindrical screw (3) with a through bore at the shank end and which is made from biodegradable, biocompatible material, characterized in that it has a flat head (32) and a hollow cylindrical shank (30) with radially outwardly directed widened portions (33) in the form of longitudinal grooves extending over the entire length of the hollow cylindrical shank and that the latter is provided with radially oriented through bores (35).

## Revendications

1. Ensemble d'implants pour ostéosynthèse permettant l'assujettissement macrostable et/ou le soutien de fragments d'os plats ou longs, en particulier chez les enfants, comprenant au moins une bande d'extension (2) qui est à même de transmettre les forces et est constituée d'une matière résorbable biocompatible, et des éléments de liaison biocompatibles (3, 10, 13) pour assujettir la bande d'extension aux fragments d'os à fixer, caractérisé en ce que la bande (2) est tricotée ou tissée de telle sorte que les fils (21) se croisent sous un angle d'environ 60° et que les fils de trame soient soudés à écart régulier avec les fils longitudinaux en sorte que la déformabilité élastique de la bande (2) soit faible dans le sens longitudinal, et permet un passage aisé lors de l'application des éléments de liaison (3, 10, 13).

2. Ensemble d'implants selon la revendication 1, caractérisé en ce que l'élément de liaison est fabriqué à partir d'une matière biodégradable et biocompatible et comprend deux parties, à savoir une douille (10) avec une partie tubulaire (11), une collerette (12) et une cheville (13) conformée en creux (15), fermée d'un côté et dotée du côté ouvert d'une collerette (16), dont la paroi externe présente des ressauts en forme de dents (14) qui sont dimensionnés de telle sorte que la cheville puisse glisser étroitement dans la partie tubulaire de la douille.

3. Ensemble d'implants selon la revendication 1, dans lequel l'élément de liaison est une vis cylindrique creuse (3) dotée d'un alésage de passage à l'extrémité tige, laquelle vis est constituée d'une matière biodégradable et biocompatible, caractérisé en ce que la vis présente une tête plate (32) et une tige cylindrique creuse (30) avec des extensions (33) sous la forme de rainures longitudinales qui sont dirigées vers l'extérieur radialement et qui s'étendent sur toute la longueur de la tige cylindrique creuse, et en ce que la tige cylindrique creuse de la vis est dotée d'alésages de passage (35) tournée radialement vers l'extérieur.

FIG. 1

FIG. 2

FIG. 5a

FIG. 5b

FIG. 5c

FIG. 3a

FIG. 3b

FIG. 4

FIG. 8

FIG.6

FIG. 7

7